(19) Europäisches Patentamt
European Patent Office
Office européen des brevets

(11) **EP 3 892 254 B1**

(12) **EUROPEAN PATENT SPECIFICATION**

(45) Date of publication and mention
of the grant of the patent:
**25.02.2026   Bulletin 2026/09**

(21) Application number: **21164633.6**

(22) Date of filing: **24.03.2021**

(51) International Patent Classification (IPC):
*A61K 6/818* (2020.01)        *A61K 6/822* (2020.01)
*A61K 6/824* (2020.01)        *A61C 13/00* (2006.01)
*A61L 27/42* (2006.01)        *C04B 35/486* (2006.01)
*C04B 35/488* (2006.01)

(52) Cooperative Patent Classification (CPC):
**A61K 6/818; A61C 13/00; A61C 13/0003;**
**A61C 13/0022; A61K 6/822; A61K 6/824;**
**A61L 27/427; C04B 35/486;** C04B 2235/3217;
C04B 2235/3224; C04B 2235/3225;
C04B 2235/3246; C04B 2235/3272;
C04B 2235/3286; C04B 2235/443;        (Cont.)

(54) **PREPARING METHOD OF ZIRCONIA MILL BLANK FOR DENTAL CUTTING AND MACHINING USING PRECIPITATE**

VERFAHREN ZUR HERSTELLUNG EINES FRÄSROHLINGS AUS ZIRKONIUMDIOXID FÜR DAS ZAHNTECHNISCHE SCHNEIDEN UND DIE BEARBEITUNG UNTER VERWENDUNG VON PRÄZIPITAT

MÉTHODE DE PRÉPARATION D'UNE ÉBAUCHE DE ZIRCONE POUR LE DÉCOUPAGE ET L'USINAGE DENTAIRE UTILISANT UN PRÉCIPITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.04.2020   JP 2020065586**

(43) Date of publication of application:
**13.10.2021   Bulletin 2021/41**

(73) Proprietor: **Shofu Inc.**
**Kyoto-shi, Kyoto 605-0983 (JP)**

(72) Inventors:
 • **NONAKA, Kazumichi**
   **KYOTO, 605-0983 (JP)**
 • **TAKAHASHI, Shuhei**
   **KYOTO, 605-0983 (JP)**

(74) Representative: **Eisenführ Speiser**
**Patentanwälte Rechtsanwälte PartGmbB**
**Postfach 31 02 60**
**80102 München (DE)**

(56) References cited:
   **US-A1- 2017 183 270      US-B2- 9 949 808**

• **WENYUAN LI ET AL: "Effect of adding urea on performance of Cu/CeO/yttria-stabilized zirconia anodes for solid oxide fuel cells prepared by impregnation method", ELECTROCHIMICA ACTA, ELSEVIER, AMSTERDAM, NL, vol. 56, no. 5, 20 November 2010 (2010-11-20), pages 2230 - 2236, XP028133052, ISSN: 0013-4686, [retrieved on 20101128], DOI: 10.1016/ J.ELECTACTA.2010.11.068**
• **ZHU X ET AL: "Fabrication and evaluation of a Ni/La"0"."7"5Sr"0"."2"5Cr"0"."5Fe"0"."5O"3"-"@d co-impregnated yttria-stabilized zirconia anode for single-chamber solid oxide fuel cells", INTERNATIONAL JOURNAL OF HYDROGEN ENERGY, ELSEVIER SCIENCE PUBLISHERS B.V., BARKING, GB, vol. 35, no. 13, 1 July 2010 (2010-07-01), pages 6897 - 6904, XP027134324, ISSN: 0360-3199, [retrieved on 20100521]**

EP 3 892 254 B1

- **SUBRT JAN: "In: Urea: Synthesis, Properties and Uses HOMOGENEOUS PRECIPITATION WITH UREA: A VERSATILE WAY TO METAL OXIDE NANOPARTICLES", 1 September 2012 (2012-09-01), XP055837314, Retrieved from the Internet <URL:https://www.researchgate.net/ publication/287246768_Homogeneous_precipita tion_with_urea_A_versatile_way_to_metal_oxid e_nanoparticles> [retrieved on 20210902]**

(52) Cooperative Patent Classification (CPC): (Cont.)
C04B 2235/449; C04B 2235/604; C04B 2235/616; C04B 2235/6562; C04B 2235/75; C04B 2235/9653; C04B 2235/9661

**Description**

BACKGROUND OF THE INVENTION

Field of the Invention

The present invention relates to a preparing method of a zirconia mill blank for dental cutting and machining.

Description of the Related Art

In recent years, techniques to prepare a prosthesis device by the cutting and machining which uses the dental CAD/CAM system spread rapidly and therefore it has been becoming possible to easily prepare prosthetic devices by cutting and machining the blanks which are made of ceramic materials such as zirconia, alumina and lithium disilicate glass, and resin materials such as an acrylic resin and a hybrid resin. In particular, zirconia has been clinically applied in various cases because of its high strength. On the other hand, since a zirconia which is fully sintered zirconia (hereinafter, referred to as "zirconia perfect sintered body") has a very high hardness, a zirconia which is calcined at a temperature lower than the perfect sintering temperature to adjust to a hardness that enables to cut and machine has been used as a zirconia mill blank for dental cutting and machining, and it is often to obtain a zirconia perfect sintered body by re-firing after cutting and machining.

Chinese Patent Application Publication No. 108472110 discloses a method for depositing a metal hydroxide in the pores of a porous zirconia molded body by treating with an $NH_4OH$ solution after impregnating the porous zirconia molded body with a solution containing metal ions. However, in this method, since the deposition proceeds from the portion in contact with the $NH_4OH$ solution, it is difficult to uniformly deposit the metal hydroxide in the pores of the porous zirconia molded body.

International Publication No. 2019/083967 discloses a method of immersing a porous zirconia molded body in a solution containing a molybdenum ion to support a molybdenum compound in the pores.

US Patent Publication No. 9,949,808 B2 describes a process for producing a ceramic body, in particular a dental ceramic blank, having selectively adjustable degrees of expression of one or more different physical properties, wherein the ceramic body has a porosity to enable the control of a selective distribution of one or more chemical substances that are suitable for influencing the physical properties of the ceramic body, and in a first step, which is a loading step, the ceramic body is loaded with one or more solutions of the one or more chemical substances. In a second step, which is a distribution step, the distribution of the one or more chemical substances within the porous ceramic body is controlled, wherein a progression and/or a spatial progression of the degree of expression of the one or more physical properties can be produced. The control is effected by adjusting one or more ambient parameters in an environment, in particular by adjusting the air humidity, the pressure and/or the temperature.

W. Li et al. (Electrochimica Acta 56 (2011) 2230-2236) describe the effect of urea on performance of Cu/-$CeO_2$/yttria-stabilized zirconia anodes for solid oxide fuel cells prepared by impregnation method.

X. Zhu et al. (International Journal of Hydrogen Energy 35 (2010) 6897-6904) describe the fabrication and evaluation of a co-impregnated yttria-stabilized zirconia anode for single-chamber solid oxide fuel cells.

J. Subrt (In: Urea: Synthesis, Properties and Uses, Chapter 3 (2012), ISBN 978-1-62257-032-4) describes homogenous precipitation with urea to obtain metal oxide nanoparticles.

However, in this method, when the molybdenum solution is dried, the molybdenum compound segregates on the surface of the porous zirconia molded body, so that it is difficult to uniformly support the molybdenum compound.

SUMMARY OF THE INVENTION

Technical Problem

It has been required to support the metal without segregation on the zirconia mill blank for dental cutting and machining which has been adjusted to a hardness that enables to cut and machine by calcining at a low temperature.

Solution to Problem

It has been found that when a preparing method of a zirconia mill blank for dental cutting and machining is according to the claims, the segregation of supported metal compound is effectively suppressed.

The precipitant is decomposed by heating in the deposition step. It is preferable that at least one of the metal ion is any one or more of an aluminum ion, a gallium ion and an indium ion. It is preferable that the precipitant is urea. The present invention also provides a zirconia mill blank for dental cutting and machining prepared by the preparing method of the present invention. The present invention also provides a dental prosthesis device prepared from the zirconia mill blank for dental cutting and machining of the present invention.

The present invention also provides a calcined zirconia mill blank for dental cutting and machining, wherein at least one of metal oxide, metal hydroxide, metal oxyhydroxide, metal carbonate, metal carbonate hydroxide and metal oxalate is deposited between zirconia particles. This zirconia mill blank for dental cutting and machining may be heat-treated. The present invention also provides a dental prosthesis device prepared from the zirconia mill blank for dental cutting and machining of the present invention.

Advantageous Effects of Invention

The preparing method of a zirconia mill blank for dental cutting and machining of the present invention can deposit a metal ion impregnated in the porous zirconia molded body as a metal compound without segregation, and therefore can provide a zirconia mill blank for dental cutting and machining in which the metal compound is uniformly supported.

DETAILED DESCRIPTION OF THE PREFERRED EMBODIMENTS

Hereinafter, specific embodiments of the present invention will be described in detail. The preparing method of a zirconia mill blank for dental cutting and machining of the present invention comprises, as a feature, an impregnation step of impregnating a porous zirconia molded body with an impregnating solution containing at least one metal ion and at least one precipitant, and a deposition step of decomposing the precipitant in the porous zirconia molded body to deposit a metal compound. The preparing method of a zirconia mill blank for dental cutting and machining of the present invention may suppress the segregation of supported metal compound, and may provide a zirconia mill blank for dental cutting and machining in which one or a plurality of metal ions are uniformly added.

As the metal ion of the present invention, any metal ion can be used without particular limitation as long as it is a metal ion that can be deposited in the pores of the porous zirconia molded body by a precipitant. Specific examples include an yttrium ion which may be used a stabilizer for zirconia, an erbium ion and an iron ion which are used as a coloring component, and an aluminum ion, a gallium ion and an indium ion which are used for the purpose of controlling sinterability, and the like.

At least one of the metal ion may be a rare earth metal ion. The specific examples of the rare earth metal ion include an yttrium ion. By containing a rare earth metal ion, it is possible to improve the translucency of the zirconia sintered body and to color the zirconia sintered body. The metal ion may be only a rare earth metal ion.

At least one of the metal ion may be a transition metal ion. The specific examples of the transition metal ion include iron ion. By containing a transition metal ion, it is possible to color the zirconia sintered body. By containing the transition metal ions, it is possible to color the zirconia sintered body. The metal ion may be only a transition metal ion.

At least one of the metal ion may be any one or more of an aluminum ion, a gallium ion and an indium ion. By containing any one or more of an aluminum ion, a gallium ion and an indium ion, it is possible to improve the sinterability of the zirconia sintered body and to improve the translucency of the zirconia sintered body. The metal ion may be only an aluminum ion, a gallium ion and/or an indium ion. The metal ion may be only a rare earth metal ion, a transition metal ion, an aluminum ion, a gallium ion and/or an indium ion.

As the precipitant of the present invention, any precipitant can be used without particular limitation as long as it can precipitate a compound containing a metal ion by decomposing the precipitant. Specific examples include urea, hexamethylene tetramine, dimethyl oxalate and the like, and it is preferable to use urea.

The metal ion source of the present invention is preferably a metal ion organic acid salt. Examples include acetates, citrates and the like.

The metal ion concentration of the impregnating solution in the present invention is not particularly limited, but in the case of an yttrium ion, for example, it is preferably about within a range of 2.0 wt.% to 10.0 wt.%. When it is 2.0 wt.% or less, there is a case that the amount of supported yttrium is small and sufficient characteristic is not obtained. When it is 10.0 wt.% or more, there is a case that the amount of supported yttrium is large and physical property is adversely affected. Since the optimum amount of supported metal depends on the type of metal and the desired characteristics, the metal ion concentration of the impregnating solution is preferably determined from the desired optimum amount of supported metal and the solubility of the metal ion source in the solvent.

The amount of the precipitant in the impregnating solution of the present invention is preferably an amount capable of depositing the entire amount of the metal ion in the impregnating solution. When the amount of the precipitant is small, there is a tendency that the time required for depositing the entire amount of the metal ion is long, and when the amount of the precipitant is large, there is a tendency that the time required for depositing the entire amount of the metal ion is short. However, in any case, there is no influence to the effect of the present invention. When the amount of the precipitant is less than the capable of depositing the entire amount of the metal ion in the impregnating solution, there is a case that the entire amount of the metal ion is not deposited and the metal ion segregates, and therefore it is not preferable. When the amount of the precipitant is excessive, there is no influence to the effect of the present invention, however, a large amount of the precipitant remains after the deposition step, and therefore it is not preferable. The amount of the precipitant is not particularly limited, but it is preferable that the amount is 5 to 30 times the amount capable of depositing the entire amount of the metal ion.

The solvent used for the impregnating solution of the present invention is not particularly limited, in the invention the boiling point is higher than the decomposition temperature of the precipitant. It is preferable to use water since it is easily available and is easy to handle.

Examples of metal compound deposited by decomposing of the precipitant in the deposition step include metal oxides, metal hydroxides, metal oxyhydroxides, metal carbonates, metal carbonate hydroxides, metal oxalates and the like, but the metal compound is not particularly limited. Moreover, the form thereof is not particularly limited. It may have any shape such as a spherical, a plate, a needle, a shapeless and the like, and may be crystalline or amorphous. By deposition of these, it is possible that the metal ion is supported on the porous zirconia molded body to prepare a zirconia mill blank for dental cutting and machining.

The impregnating solution of the present invention, may be added with acid or base, or a pH adjusting agent for the purpose of controlling the pH. The acid or base to be used is not particularly limited, but it is preferable to use an organic acid such as acetic acid, citric acid and the like or aqueous ammonia from the viewpoint of leaving no residue on the porous zirconia molded body.

It is preferable that the primary particle diameter of the zirconia powder used in preparing the porous zirconia molded body of the present invention is within a range of 1 to 500 nm. When the primary particle diameter is less than 1 nm, there is a tendency that it is difficult to impart sufficient strength, although the translucency of the zirconia sintered body is improved. On the other hand, when the primary particle diameter is more than 500 nm, there is a tendency that it is difficult to impart sufficient strength to the zirconia sintered body.

The porous zirconia molded body of the present invention may contain a stabilizer. Examples of the stabilizer include yttria, ceria, calcia, magnesia and the like.

The porous zirconia molded body in the present invention may contain a coloring material. Specific examples thereof include iron oxide for imparting a yellow color and erbium for imparting a red color. In addition, there is no problem at all even if a coloring material containing element such as cobalt, manganese and chromium for adjusting a color in addition to these coloring materials is used together. In the present disclosure, the tooth color can be easily colored by including the coloring material.

The porous zirconia molded body in the present invention may contain a sintering aid, and examples thereof include alumina.

It is preferable that a crystal phase of the porous zirconia molded body in the present invention is tetragonal and/or cubic. When the crystal phase is monoclinic phase, it is not preferable because sufficient translucency may be not imparted after perfect sintering of zirconia.

The preparing method of the porous zirconia molded body in the present invention is not particularly limited, and any known preparing methods can be used without any problem. Specifically, it is preferable to be prepared by molding a zirconia powder by a press molding.

The porous zirconia molded body in the present invention is preferably subjected to isostatic pressing by cold isostatic pressing (CIP treatment) after the press molding.

The maximum load pressure of CIP treatment in the present invention is preferably 50 Mpa or more. When the maximum load pressure is less than 50 MPa, there is a case where sufficient translucency and strength may be not imparted to the zirconia sintered body.

The holding time at the maximum load pressure of the CIP treatment in the present invention is not particularly limited, but in general, a range of 0 to 150 seconds is preferable and a range of 0 to 60 seconds is more preferable.

The time period required for the above series of processes is not particularly limited, but is usually preferably within a range of 30 seconds to 10 minutes and is more preferably within a range of 3 minutes to 7 minutes. When the time is too short, a molding body may be destroyed, and when the time is too long, production efficiency worsens, and therefore these are not preferable.

By firing the porous zirconia molded body in the present invention at a calcination temperature, the zirconia mill blank for dental cutting and machining in a sintered state is prepared. A calcination temperature of the porous zirconia molded body in the present invention is preferably within a range of 800 to 1200 °C. When the calcination temperature is less than 800 °C, because Vickers hardness and/or bending strength become too low and therefore there is a tendency that chipping and breakage easily occur in the cutting and machining. On the other hand, when the calcination temperature is more than 1200 °C, because Vickers hardness and/or bending strength become too high and therefore there is a tendency that a milling bar of a milling machine is heavily consumed to raise a running cost.

The preparing method of the zirconia powder used in preparing the porous zirconia molded body in the present invention is not particularly limited, and any known preparing methods can be used. Specifically, it is preferable that zirconia powder used in the present invention is prepared by the hydrolysis method.

As the impregnation method of impregnating the porous zirconia molded body with the impregnating solution in the present invention, any method can be used without particular limitation as long as the impregnating solution can infiltrate into the pores of the porous zirconia molded body. In a simple and preferable method, the whole and/or a part of the porous zirconia molded body is immersed in the impregnating solution. In this case, the impregnating solution can infiltrate by the capillarity into an inside of the porous zirconia molded body. The immersion time is 3 minutes or more, preferably 10 minutes to 10 hours.

The impregnating solution may be impregnated in the whole of the porous zirconia molded body and may be impregnated in an arbitrary portion of the porous zirconia molded body. The method of impregnating only an arbitrary portion is not particularly limited, and examples thereof include control of the weight and volume of the impregnating solution and control of the impregnation time.

A plurality of impregnating solutions may be used for one porous zirconia molded body. In a specific example, after impregnating the first type of impregnating solution from one end, the second type of impregnating solution is impregnated from the same end or the other end.

The atmosphere in impregnating the impregnating solution is not particularly limited, and an ambient air, an inert gas and the like can be used under normal pressure, reduced pressure, and pressurized conditions. Since no special facilities is required, it is preferable to perform under normal atmospheric pressure.

In the present invention the method of decomposing a precipitant is hydrolysis by heating and an alternative method, not part of the invention is hydrolysis by enzymes, and hydrolysis by heating is done in the invention because of its simplicity.

The method of the hydrolysis by heating is not particularly limited as long as it can maintain a constant temperature. Examples include a method of using a thermostat, a dryer, a water bath and an oil bath.

It is preferable to seal the porous zirconia molded body in order to prevent the solvent from evaporating during hydrolysis by heating. The method is not particularly limited, and examples thereof include a method of covering with a resin sheet and degassing in some cases.

The temperature of hydrolysis by heating is not particularly limited as long as it is equal to or higher than the decomposition temperature of the precipitant and it is equal to or lower than the boiling point of the solvent of the impregnating solution in the deposition step. However, when it is too low, a long heat treatment is required until the entire amount of the metal compound is precipitated, and therefore it is not preferable. Further, when the temperature exceeds the boiling point of the solvent, the solvent evaporates during the heat treatment and the supported metal compound segregates, and therefore in the invention, the precipitant is decomposed by heating at a temperature which is equal to or lower than the boiling point of the solvent of the impregnating solution in the deposition step.

The time required for hydrolysis by heating depends on the metal ion concentration, the precipitant concentration, the heat treatment temperature and the like, and therefore it is necessary to be adjusted appropriately, but it is generally about 10 minutes to several days. It is preferably 10 minutes to 24 hours.

It is preferable to comprise a drying step after the deposition step. The drying step is a step of drying the porous zirconia molded body in which the metal compound is deposited. It is preferable to dry in a dryer in the drying step. It is preferable that the drying temperature is within a range of 90 to 200 °C, and the drying time is within a range of 15 minutes to 5 hours. It is preferable to sufficiently vaporize the solvent after drying. In some cases, the heat treatment step may be carried out following this drying step. The drying step may also be included in the heat treatment step.

Since the solvent, unreacted product, by-product and the like remain in the porous zirconia molded body in which the metal compound is deposited and supported, prepared in such a way, it is preferable to perform a heat treatment. The heat treatment method is not particularly limited, but heat treatment under normal atmospheric pressure is preferable since no special facilities is required. The heat treatment temperature is not particularly limited, but it is preferably within a range of 500 °C to 1200 °C. By heat treatment, impurities, organic substances and odors can be removed.

In this way, a zirconia mill blank for dental cutting and machining can be prepared by the preparing method of the present invention. It is preferable that the prepared zirconia mill blank for dental cutting and machining is severed, cut, and polished so as to have a desired size as necessary.

The method for perfect sintering the zirconia mill blank for dental cutting and machining of the present invention to prepare a prosthesis device in a sintered state is not particularly limited, but a simple and preferable method is to firing at normal pressure. The firing temperature is not particularly limited, but is preferably within a range of 1400 to 1600 °C, more preferably within a range of 1450 to 1550 °C. The holding time at the maximum firing temperature is not particularly limited, but is preferably within a range of 1 minute to 12 hours, and more preferably within a range of 2 to 4 hours. The temperature increase rate is not particularly limited, but is preferably within a range of 1 to 400 °C/min, and more preferably within a range of 3 to 100 °C/h.

The kind of a prosthesis device prepared by cutting and machining the zirconia mill blank for dental cutting and machining according to the present invention is not limited particularly, and there is no problem at all even if the prosthesis device is any of an inlay, a laminate, a crown, a bridge and the like. Therefore, a shape of a zirconia mill blank for dental cutting and machining which is cut and machined for preparing a prosthesis device is not limited particularly, and any zirconia mill blank for dental cutting and machining can be used even if the zirconia mill blank for dental cutting and machining has any shape such as a block shape corresponding to an inlay, a laminate, a crown and the like and a disk shape corresponding to a bridge.

In order to prepare the zirconia mill blank for dental cutting and machining of the present invention, it is preferable to fire the porous zirconia molded body at least once at a calcination temperature within a range of 800 to 1200 °C. The heat treatment temperature of the porous zirconia molded body in which the metal compound is deposited and supported is preferably within a range of 500 °C to 1200 °C. In this heat treatment, when the temperature exceeds the above calcination temperature, the state may be defined as the sintered state of the zirconia mill blank for dental cutting and machining.

In the present invention, a prosthesis device can be prepared by cutting and machining the zirconia mill blank for dental cutting and machining and by firing at 1400 °C to 1600 °C. In the present invention, a prosthesis device can be prepared by firing the zirconia mill blank for dental cutting and machining at 1400 °C to 1600 °C without cutting and machining to prepare a zirconia mill blank for dental cutting and machining with a sintered state of the prosthesis device and then by cutting and machining. It is preferable that the relative density of the prosthesis device and the zirconia mill blank for dental cutting and machining with a sintered state of the prosthesis device is 98% or more of the theoretical density. The relative density is determined by the measured density/the theoretical density. When the relative density is less than 98%, the strength and translucency tend to be lowered.

[Examples]

Hereinafter, the present invention is described by way of Examples in more detail, and specifically, but the present invention is not limited to these Examples.

(Preparation of porous zirconia molded body)

Zirconia powder containing 2.0 mol% of solid-solved yttrium oxide was filled in a mold ($\varphi$100 mm), and press molding (surface pressure: 50 MPa) was performed to obtain a molded body. Further, the molded body was subjected to CIP treatment (maximum load pressure: 200 MPa, holding period: 1 minute. Thereafter, calcination was performed in an electric furnace (1000 °C, 30 minutes) to prepare a porous zirconia molded body.

(Preparation of impregnating solution)

Table 1 shows the composition of the impregnating solution. According to the composition shown in Table 1, 1000 g of the impregnating solution was prepared by adding a metal salt and a precipitant to ion-exchanged water and stirring for 1 hour.

[Table 1]

|  | Solution 1 | Solution 2 | Solution 3 | Solution 4 | Solution 5 |
|---|---|---|---|---|---|
| Solvent | Ion-exchanged water | Ion-exchanged water | Ion-exchanged water | Ion-exchanged water | Ion-exchanged water |
| Metal salt | Yttrium acetate tetrahydrate | Erbium acetate tetrahydrate | Iron(III) Nitrate Nonahydrate | Gallium(III) Nitrate n-Hydrate | Aluminium Nitrate Nonahydrate |
| Metal salt concentration(wt%) | 10 | 10 | 20 | 10 | 20 |
| Precipitant | Urea | Urea | Urea | Urea | Urea |
| Amount of precipitant(mol)/ Amount of metal ions(mol) | 20 | 20 | 10 | 15 | 5 |

|  | Solution 6 | Solution 7 | Solution 8 | Solution 9 | Solution 10 |
|---|---|---|---|---|---|
| Solvent | Ion-exchanged water | Ion-exchanged water | Ion-exchanged water | Ion-exchanged water | Ion-exchanged water |
| Metal salt | Yttrium acetate tetrahydrate | Yttrium acetate tetrahydrate | Yttrium acetate tetrahydrate | Yttrium acetate tetrahydrate | Erbium acetate tetrahydrate |
| Metal salt concentration(wt%) | 10 | 7 | 5 | 10 | 10 |
| Precipitant | Hexamethylenetetramine | Urea | Urea | – | – |
| Amount of precipitant(mol)/ Amount of metal ions(mol) | 8 | 20 | 20 | 0 | 0 |

|  | Solution 11 | Solution 12 | Solution 13 | Solution 14 |
|---|---|---|---|---|
| Solvent | Ion-exchanged water | Ion-exchanged water | Ion-exchanged water | Ion-exchanged water |
| Metal salt | Iron(III) Nitrate Nonahydrate | Gallium(III) Nitrate n-Hydrate | Aluminium Nitrate Nonahydrate | Yttrium tetrahydrate acetate |
| Metal salt concentration(wt%) | 20 | 10 | 20 | 10 |
| Precipitant | – | – | – | Urea |
| Amount of precipitant(mol)/ Amount of metal ions(mol) | 0 | 0 | 0 | 20 |
| pH adjusting agent | – | – | – | Acetic acid |
| pH adjusting agent concentration(wt%) | – | – | – | 2 |

(Supporting a metal compound on a porous zirconia molded body)

The porous zirconia molded body was placed in a container, and the impregnating solution was poured therein so that only the top surface of the porous zirconia molded body was exposed, and then left standing under normal atmospheric pressure atmosphere for 24 hours. Thereafter, the porous zirconia molded body was taken out from the impregnating solution, and after removing the excess impregnating solution, it was placed in a resin bag and degassed. The porous zirconia molded body placed in a resin bag and degassed was placed in a dryer, and then a heat treatment at 95 °C for 15 hours was performed to deposit a metal compound. After the heat treatment, the porous zirconia molded body was taken out from the resin bag and dried (120 °C, 1 hour) to prepare a porous zirconia molded body supporting a metal compound. This porous zirconia molded body was heat treated in an electric furnace (500 °C, 30 minutes) to prepare a zirconia mill blank for dental cutting and machining.

(Measurement of amount of supported metal (wt.%))

The five test specimens for evaluating the amount of supported metal was prepared by cutting and machining the zirconia mill blank for dental cutting and machining into a round plate shape ($\varphi$24 mm $\times$ 1.6 mm). The amount of metal on each of the upper surface and the lower surface of each test specimen was measured by using a fluorescent X-ray analysis device (manufactured by Rigaku Corporation). Further, the amount of supported metal (wt.%) is shown in terms of all oxide. Furthermore, since the used zirconia powder contained a certain amount of yttrium and aluminum, when the supported metal was yttrium and aluminum, the value by subtracting the amount contained in the zirconia powder from the measured value was shown as the supported amount.

(Evaluation of variation in amount of supported metal (wt.%))

The amount of metal on the upper and lower surfaces of each of the five test specimen measured in "Measurement of amount of supported metal (wt.%)" was set as the measured value at 10 points in each zirconia mill blank for dental cutting and machining, and "the maximum value-minimum value" and "standard deviation" in these measured values were calculated.

Table 2 shows the characteristic test results of the prepared zirconia mill blank for dental cutting and machining in Examples and Comparative Examples.

(Color Measurement)

The color measurement test was performed on Example 1, Example 3, Comparative Example 1 and Comparative Example 3. The color measurement test specimen was prepared from the test specimen for evaluation of amount of supported metal. Each test specimen for evaluation of amount of supported metal was perfect sintered (firing temperature: 1550 °C, temperature increase rate: 5 °C/min, holding time: 2 hours) in a firing furnace. Then, the thickness (1.0 mm) of each test body was adjusted with a surface grinder. The Color measurement was performed by using a spectrocolorimeter (manufactured by Konica Minolta). From the obtained data, $\Delta E^*$ was calculated by the following formula. Table 3 shows the color measurement test results.

$$\Delta E^* = [(L^*_a - L^*_b)^2 + (a^*_a - a^*_b)^2 + (b^*_a - b^*_b)^2]^{1/2}$$

(Evaluation of Translucency)

The evaluation of translucency was performed on Example 1, Example 3, Comparative Example 1 and Comparative Example 3. The translucency was evaluated by measuring the contrast ratio. The contrast ratio was measured by using a spectrocolorimeter (manufactured by Konica Minolta). In the following formula, Yw is the value Y measured by placing the white plate behind the test specimen, and Yb is the value Y measured by placing the black plate behind the test specimen. The contrast ratio was calculated from the following formula. When the contrast ratio value is close to zero, the materials are transparency. When the contrast ratio value is close to 1, the materials are opaqueness. The evaluation results of translucency are shown in Table 3.

$$\text{Formula: The contrast ratio} = Yb / Yw$$

[Table 2]

| | Example 1 | Example 2 | Example 3 | Example 4 | Example 5 | Example 6 | Example 7 | Example 8 | Example 9 | Example 10* |
|---|---|---|---|---|---|---|---|---|---|---|
| Solution | Solution 1 | Solution 2 | Solution 3 | Solution 4 | Solution 5 | Solution 6 | Solution 7 | Solution 8 | Solution 14 | Solution 1 |
| Supported metal | Y | Er | Fe | Ga | Al | Y | Y | Y | Y | Y |
| Supported metal concentration (Max) (wt%) | 0.62 | 1.17 | 0.74 | 0.51 | 0.55 | 0.64 | 0.42 | 0.31 | 0.61 | 0.63 |
| Supported metal concentration (Min) (wt%) | 0.53 | 1.10 | 0.67 | 0.45 | 0.47 | 0.52 | 0.32 | 0.24 | 0.51 | 0.53 |
| Maximum-minimum (wt%) | 0.09 | 0.07 | 0.07 | 0.06 | 0.08 | 0.12 | 0.10 | 0.07 | 0.10 | 0.10 |
| Standard deviation | 0.03 | 0.02 | 0.03 | 0.02 | 0.03 | 0.04 | 0.04 | 0.03 | 0.03 | 0.03 |

*Containing 1.2 wt% iron as a colorant

| | Comparative example 1 | Comparative example 2 | Comparative example 3 | Comparative example 4 | Comparative example 5 |
|---|---|---|---|---|---|
| Solution | Solution 9 | Solution 10 | Solution 11 | Solution 12 | Solution 13 |
| Supported metal | Y | Er | Fe | Ga | Al |
| Supported metal concentration (Max) (wt%) | 3.45 | 4.52 | 3.52 | 3.13 | 3.49 |
| Supported metal concentration (Min) (wt%) | 0.28 | 0.73 | 0.49 | 0.23 | 0.47 |
| Maximum-minimum (wt%) | 3.17 | 3.79 | 3.03 | 2.90 | 3.02 |
| Standard deviation | 1.49 | 1.74 | 1.39 | 1.33 | 1.42 |

[Table 3]

| | Example 1 | | Comparative example 1 | |
|---|---|---|---|---|
| Solution | Solution 1 | | Solution 9 | |
| Supported metal | Y | | Y | |
| Supported metal concentration | Max | Min | Max | Min |
| Contrast ratio | 0.65 | 0.65 | 0.73 | 0.69 |
| Ratio of Contrast ratio(Max / Min) | 1.00 | | 1.05 | |

| | Example 3 | | Comparative example 3 | |
|---|---|---|---|---|
| Solution | Solution 3 | | Solution 11 | |
| Supported metal | Fe | | Fe | |
| Supported metal concentration | Max | Min | Max | Min |
| L＊ | 65.93 | 66.03 | 57.22 | 74.85 |
| a＊ | 5.80 | 3.12 | 11.98 | -3.80 |
| b＊ | 27.28 | 27.51 | 23.76 | 26.14 |
| ΔE＊ | 2.69 | | 23.8 | |

In Example 1, since the impregnating solution contained a precipitant and the metal compound was supported by the deposition due to the decomposition of the precipitant, a zirconia mill blank for dental cutting and machining which had little variation in the amount of the supported metal depending on the portions and had little variation in the translucency after perfect sintering was obtained.

In Example 2, since the impregnating solution contained a precipitant and the metal compound was supported by the deposition due to the decomposition of the precipitant, a zirconia mill blank for dental cutting and machining which had little variation in the amount of the supported metal depending on the portions was obtained.

In Example 3, since the impregnating solution contained a precipitant and the metal compound was supported by the deposition due to the decomposition of the precipitant, a zirconia mill blank for dental cutting and machining which had little variation in the amount of the supported metal depending on the portions and had little variation in the color tone after perfect sintering was obtained.

In Examples 4 to 8, since the impregnating solution contained a precipitant and the metal compound was supported by the deposition due to the decomposition of the precipitant, a zirconia mill blank for dental cutting and machining which had little variation in the amount of the supported metal depending on the portions was obtained.

In Comparative Example 1, since the impregnating solution did not contain a precipitant and the metal compound was supported by the evaporation of the solvent in the drying step, a zirconia mill blank for dental cutting and machining which had large variation in the amount of the supported metal depending on the portion and had large variation in the translucency after perfect sintering was obtained. In general, there is a tendency that the portion having a large amount of yttrium has higher translucency, but in the portion having maximum amount of the supported metal in Comparative Example 1, it was confirmed that the translucency decreased because the amount of supported yttrium was too large.

In Comparative Example 2, since the impregnating solution did not contain a precipitant and the metal compound was supported by the evaporation of the solvent in the drying step, a zirconia mill blank for dental cutting and machining which had large variation in the amount of the supported metal depending on the portion was obtained.

In Comparative Example 3, since the impregnating solution did not contain a precipitant and the metal compound was supported by the evaporation of the solvent in the drying step, a zirconia mill blank for dental cutting and machining which had large variation in the amount of the supported metal depending on the portion and had large variation in the color tone after perfect sintering was obtained.

In Comparative Examples 4 and 5, since the impregnating solution did not contain a precipitant and the metal compound was supported by the evaporation of the solvent in the drying step, a zirconia mill blank for dental cutting and machining which had large variation in the amount of the supported metal depending on the portion was obtained.

[Industrial applicability]

The present invention relates to an invention of a zirconia mill blank for dental cutting and machining and a preparing method thereof, and is a technique which can be used in the dental field.

**Claims**

1. A preparing method of a zirconia mill blank for dental cutting and machining, comprising

   an impregnation step of impregnating a porous zirconia molded body with an impregnating solution containing at least one metal ion and at least one precipitant, and
   a deposition step of decomposing the precipitant in the porous zirconia molded body to deposit a metal compound wherein
   the precipitant is decomposed by heating at a temperature which is equal to or lower than the boiling point of the solvent of the impregnating solution in the deposition step.

2. The preparing method of a zirconia mill blank for dental cutting and machining according to claim 1, wherein at least one of the metal ion is a rare earth metal ion.

3. The preparing method of a zirconia mill blank for dental cutting and machining according to claim 1 or 2, wherein at least one of the metal ion is a transition metal ion.

4. The preparing method of a zirconia mill blank for dental cutting and machining according to any one of claims 1 to 3, wherein
   at least one of the metal ion is any one or more of an aluminum ion, a gallium ion and an indium ion.

5. The preparing method of a zirconia mill blank for dental cutting and machining according to any one of claims 1 to 4, wherein
   the precipitant is urea.

6. A zirconia mill blank for dental cutting and machining prepared by the preparing method of a zirconia mill blank for dental cutting and machining according to any one of claims 1 to 5.

7. A dental prosthesis device prepared from the zirconia mill blank for dental cutting and machining according to claim 6.

8. The preparing method of a zirconia mill blank for dental cutting and machining according to claim 1, further comprising heat treating the zirconia mill blank for dental cutting and machining at 800 to 1200 °C.

**Patentansprüche**

1. Herstellungsverfahren eines Zirkonia-Fräsrohlings für das dentale Schneiden und Bearbeiten, umfassend

   einen Imprägnierungsschritt, in dem ein poröser Zirkonia-Formkörper mit einer Imprägnierlösung imprägniert wird, die mindestens ein Metallion und mindestens einen Fällungsstoff enthält, und
   einen Abscheidungsschritt, in dem der Fällungsstoff in dem porösen Zirkonia-Formkörper zersetzt wird, um eine Metallverbindung abzuscheiden, wobei
   der Fällungsstoff durch Erhitzen bei einer Temperatur zersetzt wird, die gleich oder niedriger ist als der Siedepunkt des Lösungsmittels der Imprägnierlösung im Abscheidungsschritt.

**2.** Herstellungsverfahren eines Zirkonia-Fräsrohlings für das dentale Schneiden und Bearbeiten nach Anspruch 1, wobei

mindestens eines des mindestens einen Metallions ein Seltenerd-Metallion ist.

**3.** Herstellungsverfahren eines Zirkonia-Fräsrohlings für das dentale Schneiden und Bearbeiten nach Anspruch 1 oder 2, wobei

mindestens eines des mindestens einen Metallions ein Übergangsmetall-Ion ist.

**4.** Herstellungsverfahren eines Zirkonia-Fräsrohlings für das dentale Schneiden und Bearbeiten nach einem der Ansprüche 1 bis 3, wobei

mindestens eines des mindestens einen Metallions eines oder mehrere aus Aluminium-Ion, Gallium-Ion und Indium-Ion ist.

**5.** Herstellungsverfahren eines Zirkonia-Fräsrohlings für das dentale Schneiden und Bearbeiten nach einem der Ansprüche 1 bis 4, wobei

der Fällungsstoff Harnstoff ist.

**6.** Zirkonia-Fräsrohling für das dentale Schneiden und Bearbeiten, hergestellt durch das Herstellungsverfahren eines Zirkonia-Fräsrohlings für das dentale Schneiden und Bearbeiten nach einem der Ansprüche 1 bis 5.

**7.** Dentale Prothesenvorrichtung, hergestellt aus dem Zirkonia-Fräsrohling für das dentale Schneiden und Bearbeiten nach Anspruch 6.

**8.** Herstellungsverfahren eines Zirkonia-Fräsrohlings für das dentale Schneiden und Bearbeiten nach Anspruch 1, weiterhin umfassend

eine Wärmebehandlung des Zirkonia-Fräsrohlings für das dentale Schneiden und Bearbeiten bei 800 bis 1200 °C.

**Revendications**

**1.** Procédé de préparation d'une ébauche de fraisage en zircone pour la découpe et l'usinage dentaires, comprenant

une étape d'imprégnation consistant à imprégner un corps moulé en zircone poreuse avec une solution d'imprégnation contenant au moins un ion métallique et au moins un agent précipitant; et
une étape de dépôt consistant à décomposer l'agent précipitant dans le corps moulé en zircone poreuse afin de déposer un composé métallique, dans lequel
l'agent précipitant est décomposé par chauffage à une température qui est inférieure ou égale au point d'ébullition du solvant de la solution d'imprégnation lors de l'étape de dépôt.

**2.** Procédé de préparation d'une ébauche de fraisage en zircone pour la découpe et l'usinage dentaires selon la revendication 1, dans lequel

au moins un dudit au moins un ion métallique est un ion métallique de terre rare.

**3.** Procédé de préparation d'une ébauche de fraisage en zircone pour la découpe et l'usinage dentaires selon la revendication 1 ou 2, dans lequel

au moins un dudit au moins un ion métallique est un ion de métal de transition.

**4.** Procédé de préparation d'une ébauche de fraisage en zircone pour la découpe et l'usinage dentaires selon l'une quelconque des revendications 1 à 3, dans lequel

au moins un dudit au moins un ion métallique est un ou plusieurs parmi un ion d'aluminium, un ion de gallium et un ion d'indium.

**5.** Procédé de préparation d'une ébauche de fraisage en zircone pour la découpe et l'usinage dentaires selon l'une quelconque des revendications 1 à 4, dans lequel

ledit agent précipitant est l'urée.

**6.** Ebauche de fraisage en zircone pour la découpe et l'usinage dentaires, préparée par le procédé de préparation d'une ébauche de fraisage en zircone pour la découpe et l'usinage dentaires selon l'une quelconque des revendications 1 à 5.

**7.** Dispositif de prothèse dentaire préparé à partir de l'ébauche de fraisage en zircone pour la découpe et l'usinage dentaires selon la revendication 6.

**8.** Procédé de préparation d'une ébauche de fraisage en zircone pour la découpe et l'usinage dentaires selon la revendication 1, comprenant en outre
un traitement thermique de l'ébauche de fraisage en zircone pour la découpe et l'usinage dentaires, entre 800 et 1200 °C.

**REFERENCES CITED IN THE DESCRIPTION**

**Patent documents cited in the description**

- CN 108472110
- US 2019083967 A
- US 9949808 B2

**Non-patent literature cited in the description**

- **W. LI et al.** *Electrochimica Acta*, 2011, vol. 56, 2230-2236
- **X. ZHU et al.** *International Journal of Hydrogen Energy*, 2010, vol. 35, 6897-6904
- **J. SUBRT**. Urea: Synthesis, Properties and Uses. 2012